(19) Europäisches Patentamt
European Patent Office
Office européen des brevets

(11) Publication number: 0 189 322
A2

(12) EUROPEAN PATENT APPLICATION

(21) Application number: 86300448.7

(22) Date of filing: 23.01.86

(51) Int. Cl.⁴: C 12 P 19/40, C 12 N 9/10

(30) Priority: 25.01.85 US 695040

(43) Date of publication of application: 30.07.86
Bulletin 86/31

(84) Designated Contracting States: AT BE CH DE FR GB IT LI NL SE

(71) Applicant: ELI LILLY AND COMPANY, Lilly Corporate Center, Indianapolis Indiana 46285 (US)

(72) Inventor: Bowsher, Ronald Reed, 70 South 12th Avenue, Beech Grove Indiana 46017 (US)
Inventor: Henry, David Patterson, II, 2266 Wynnedale Road, Indianapolis Indiana 46208 (US)

(74) Representative: Hudson, Christopher Mark et al, Erl Wood Manor, Windlesham Surrey GU20 6PH (GB)

(54) **Enzymatic synthesis of S-adenosylmethionine.**

(57) The present invention provides an improved process for enzymatically preparing purified S-adenosylmethionine employing substantially purified methionine adenosyltransferase as the enzyme catalyst. The S-adenosylmethionine thus prepared is ideally suited for use in radioenzymatic assays.

EP 0 189 322 A2

# ENZYMATIC SYNTHESIS OF S-ADENOSYLMETHIONINE

While amino acids are known for their ability to serve as building blocks for proteins, they also serve an important metabolic role as precursors to a variety of biochemical compounds such as hormones and neurotransmitters. For example, the methyl group of the amino acid methionine is utilized for the formation of a large number of methylated biochemicals. In order for methionine to be used as a methyl donor, it must first be activated with adenosine 5'-triphosphate (ATP) to afford S-adenosylmethionine (SAM). A major use of radiolabeled S-adenosylmethionine is in the quantification of various endogenous compounds, such as the biogenic amines norepinephrine and histamine. For this reason radiolabeled S-adenosylmethionine is an important radiochemical.

Aberrations of the sympathetic nervous system can lead to a wide variety of adverse clinical manifestations. Therefore, accurate and reliable methods for quantifying the concentration of various compounds such as neurohormones in the body are critical to provide adequate monitoring of the system. Further, since these compounds are present in the body in very small amounts, the methods must be highly sensitive, that is, capable of detecting the compounds in very small amounts. The frequency at which these methods are conducted mandate further that they be highly reproducible under laboratory conditions when analyzing a variety of body tissues and fluids and that they provide results quickly in order to facilitate diagnosis.

Radioenzymatic assays are sensitive analytical methods which have found wide use in the quantification of various biogenic amines. These assays are based on the enzymatic methylation of a specified compound to a radiolabeled product by an appropriate enzyme employing radioactive S-adenosylmethionine as the methyl donor. Many of the currently employed radioenzymatic assays lack the sensitivity necessary to quantify catecholamines in important biological samples such as human plasma.

It has been determined that commercially available radiolabeled S-adenosylmethionine contains a number of impurities which detract from the sensitivity of any radioenzymatic assay in which it is used. The general synthetic approach used by manufacturers of this compound is to use relatively impure but easily producible preparations of methionine adenosyltransferase (MAT), the enzyme used in the enzymatic synthesis of S-adenosylmethionine. Consequently, the radiolabeled S-adenosylmethionine product requires extensive purification following its synthesis in order to remove radiolabeled contaminants and to make the S-adenosylmethionine suitable for use in radioenzymatic assays. A typical procedure currently used to prepare and purify S-adenosylmethionine is illustrated by Cantoni in Biochemical Preparations 5, 58 (1955) and METHODS IN ENZYMOLOGY 2, 254, Academic Press, NY (1955). A process for further purification of S-adenosylmethionine is described in R.L. Glazer and A.L. Peale, Analytical Biochem, 91, 516 (1978).

The present invention relates to an improved method for preparing highly purified S-adenosylmethionine. It has been discovered that crude preparations of methionine adenosyltransferase catalyze the formation of radiolabeled impurities during the enzymatic synthesis of radiolabeled S-adenosylmethionine that limit the utility of radiolabeled S-adenosylmethionine in radioenzymatic assays. Accordingly, the present method relates to the enzymatic preparation of S-adenosylmethionine employing substantially purified methionine adenosyltransferase.

The present invention relates to an improved process for the enzymatic synthesis of S-adenosylmethionine wherein L-methionine is reacted with at least a molar equivalent amount of adenosine 5'-triphosphate in the presence of a catalytic amount of methionine adenosyltransferase in a suitable buffer at a temperature in the range of about 20°C to about 40°C, a substantially purified methionine adenosyltransferase preparation being used as the source of methionine adenosyltransferase.

The invention also provides a substantially purified methionine adenosyltransferase preparation and a process for preparing it.

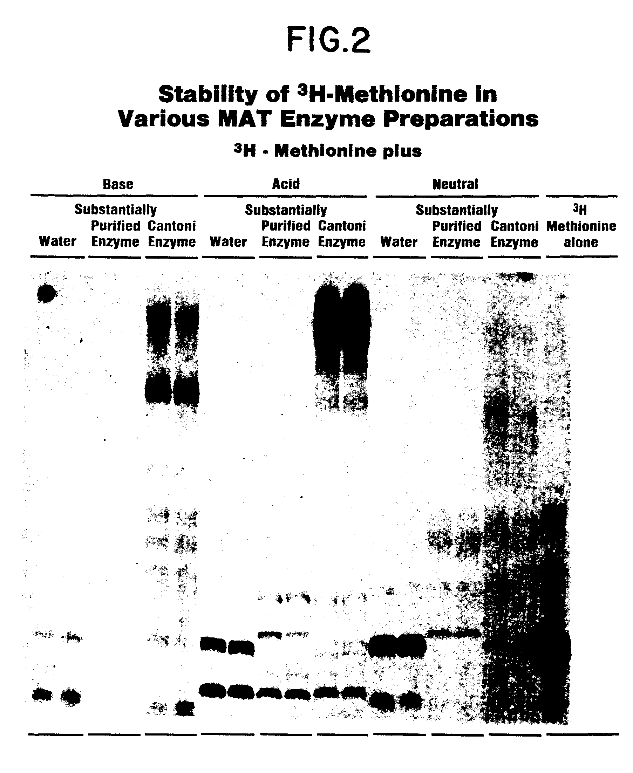

Figures 1 and 2 are autoradiograms.

Figure 1 represents a comparison of the purity of the substantially purified enzyme methionine adenosyltransferase (MAT) employed in the present process and that purified by the procedure of <u>Cantoni</u> in <u>Biochemical Preparations</u> 5, 58 (1955). The results in the figure

were obtained by incubating each enzyme separately with tritiated S-adenosylmethionine for 60 minutes at 37°C. The figure illustrates the significant improvement in purity of the enzyme used in the present process as compared to that of Cantoni.

Figure 2 again provides a comparison of methionine adenosyltransferase employed in the present process and the enzyme purified by the procedure of Cantoni, supra. The results in the figure were obtained by incubating each enzyme separately with tritiated L-methionine at about 37°C for 30 minutes. Again, the improved purity of the enzyme employed in the present process is apparent since there are a fewer number of radiolabeled contaminants.

The following scheme represents the biochemical procedure employed in the present enzymatic synthesis of S-adenosylmethionine:

L-methionine
methionine adenosyltransferase
S-adenosylmethionine
ATP
$PP_i + P_i$

wherein ATP is adenosine 5'-triphosphate, $PP_i$ is inorganic pyrophosphate and $P_i$ is inorganic phosphate.

The enzyme used to catalyze the synthesis of L-methionine to S-adenosylmethionine is known as either S-adenosylmethionine synthetase or methionine adenosyltransferase, the latter of which will be used herein. We have discovered that by employing substantially purified enzyme in the synthesis of radiolabeled S-adenosylmethionine, a dramatic improvement in the purity of the

product is obtained, resulting in a preparation particularly well suited for use in radioenzymatic assays.

While the present method may be used to prepare unlabeled S-adenosylmethionine, the procedure is preferably employed to synthesize radiolabeled material. As such, the term "S-adenosylmethionine", as used herein, represents both radiolabeled and unlabeled material. Radiolabeled S-adenosylmethionine will typically have either a tritium label on the donor methyl group, the compound known as S-[methyl-$^{3}$H]-adenosyl-L-methionine, or a [$^{14}$C]carbon atom on the donor methyl group, the compound known as S-[methyl-$^{14}$C]-adenosyl-L-methionine. These radiolabeled methyl donors may be represented by the following structures:

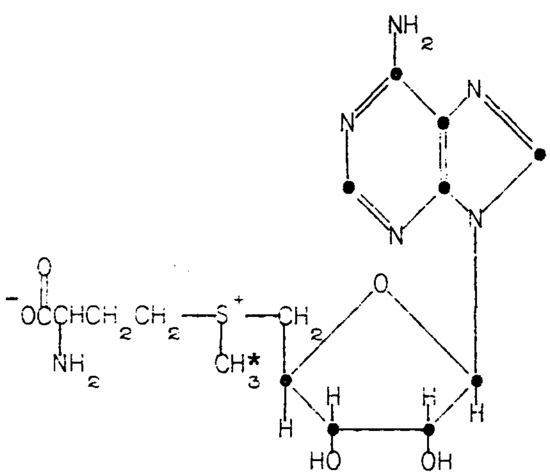

S-[methyl-$^{3}$H]-adenosyl-L-methionine

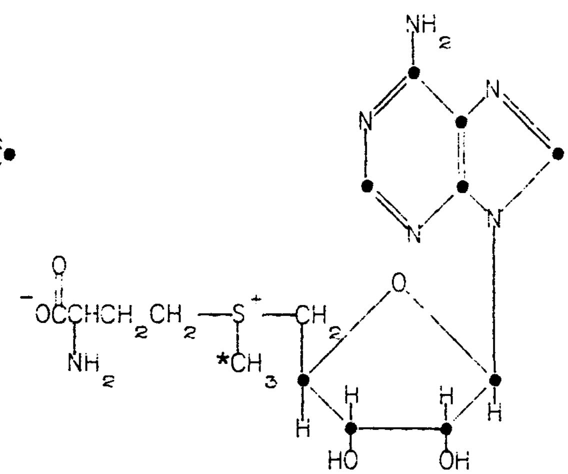

S-[methyl-$^{14}$C]-adenosyl-L-methionine

wherein the asterisk (*) represents the position of the radiolabel.

The present process is carried out by combining one mole equivalent of L-methionine with at least one mole equivalent of ATP and a catalytic amount of substantially purified methionine adenosyltransferase in a suitable buffer. The enzymatic preparation of S-adenosylmethionine with impure enzyme is well known in the art and these general conditions may be used in the present process as well. The present invention lies in the use of substantially purified methionine adenosyltransferase in the process and the recognition that the enzyme used previously was of such impurity as to create S-adenosylmethionine unacceptable for most uses where sensitivity is critical, such as in radioenzymatic assays.

Suitable buffers for use herein are well known and will typically have a useful pH in the range of about 7.0 to about 8.5 with the capacity not to chelate to magnesium. Exemplary buffers of this type include 1,3-bis[tris(hydroxymethyl)methylamino]propane (bis-tris propane), bis[2-hydroxyethyl]amino-tris[hydroxymethyl]-methane (bis-tris), and N-tris[hydroxymethyl]methyl-2-aminoethanesulfonic acid (TES).

The term "substantially purified methionine adenosyltransferase", as used herein, is defined as enzyme having a composition essentially free from foreign contaminants and of sufficient purity so as to be suitable for use in the synthesis of radiolabeled S-adenosylmethionine of sufficient purity for use in radioenzymatic assays. It has been determined experimentally that the purity of enzyme used in the present enzymatic synthesis is critical in affording S-adenosylmethionine of sufficient purity. In particular, the removal of methyltransferase enzymes and small molecular weight endogenous compounds is pivotal to the synthesis of high purity S-adenosylmethionine.

If methyl transferases are not removed from methionine adenosyltransferase (MAT), and the MAT is used to synthesize S-adenosylmethionine (SAM), then the methyl transferases can catalyze undesirable reactions with part of the SAM. This results in radiolabelled impurities in the remaining SAM that are difficult to remove. Similarly, if small molecular weight endogenous compounds that are susceptible to methylation by SAM are not removed from MAT before it is used in synthesizing SAM, then part of the SAM may react with these impurities.

Mammalian methyl transferases typically have molecular weights of 25,000 to 30,000. Biogenic amines of interest typically have molecular weights around 300. Methionine adenosyltransferase has a molecular weight on the order of 80,000. Accordingly, removal of impurities having molecular weights below 30,000 (and preferably all those having molecular weights below 50,000) from a methionine adenosyltransferase preparation, which can be accomplished using size exclusion chromatography, provides a substantially purified methionine adenosyltransferase preparation that is essentially free of biogenic amines and methyl transferases.

A catalytic amount of substantially purified methionine adenosyltransferase is used in the present process. The exact quantity of enzyme employed in the

process differs depending on its specific activity. As one of ordinary skill in the art would understand, enzyme activity is determined pragmatically by titrating the substantially purified enzyme according to standard procedures. While it is preferable to use an excess of enzyme in the process, especially when synthesizing radiolabeled S-adenosylmethionine since L-methionine is an expensive reagent, a quantity of enzyme which gives maximal conversion of substrate to product should be used.

The following procedure illustrates the method used for obtaining substantially purified methionine adenosyltransferase suitable for use in the present process. This procedure is designed to remove competing methyltransferase enzymes and naturally occuring substrates which are believed to exhaust and compete for available S-adenosylmethionine in the synthetic process and in radioenzymatic assays.

Methionine adenosyltransferase is localized almost exclusively in the liver of mammals, with lesser amounts of the enzyme in several other tissues. Typical sources of liver containing enzyme include rabbits and especially rats.

The mammalian tissue containing enzyme is isolated by standard procedures and immediately chilled, for example by submersion in an isotonic solution of sodium chloride. When used, the temperature of this solution is maintained in the range of about 0°C to about 5°C. This temperature range is also employed for all subsequent steps of the enzyme purification process.

The mammalian tissue thus isolated must be disrupted in order to facilitate extraction of the enzyme. Tissue disruption may be conducted mechanically by any of several well known procedures such as sonication, by means of a tissue press or preferably by homogenization. Homogenization may be conducted by any one of several routine procedures but is preferably carried out by first mincing the enzyme containing tissue into small pieces and then combining these pieces with an isotonic media in an homogenizer. Homogenizers suitable for use herein include blenders and other instruments, such as a Brinkmann Polytron. Suitable isotonic media include sucrose, magnesium chloride or a phosphate buffer such as sodium phosphate or potassium phosphate. The preferred isotonic media is an isotonic potassium chloride solution containing magnesium chloride. While the amount of isotonic media employed should be sufficient to completely solubilize the enzyme, the isotonic media is preferably employed at a volume of approximately 3 to 10 times the volume of the mammalian tissue sample.

The tissue suspension is then centrifuged for a period of about 15 to 60 minutes at a force in the range of about 10,000 x g to about 60,000 x g. Centrifugation is preferably conducted for about 20 minutes at a force of about 40000 x g. The concentration of the mixture is adjusted to approximately 1 mM by the addition of dithioerythritol (DTE). The filtered supernatant is then typically centrifuged at a force of about 100,000 x g to 300,000 x g for a period of about 15 to

about 120 minutes so as to remove high molecular weight contaminants. Centrifugation is preferably conducted at a force of about 220,000 x g for a period of approximately 60 minutes.

The supernatant thus formed is then filtered, typically through gauze, so as to remove the lipid layer. The supernatant thus prepared is isolated and slurried with solid enzyme-grade ammonium sulfate which causes the enzyme to precipitate. The purpose of the ammonium sulfate precipitation is to remove any small molecules, such as proteins, and to simultaneously concentrate the enzyme preparation. The ammonium sulfate is employed at a concentration in the range of about 55% to about 85%, more preferably at a concentration to provide about a 65% saturated solution of ammonium sulfate (413 g of ammonium sulfate for each 1000 ml of enzyme preparation). The preparation is stirred for a period of about 5 to about 60 minutes, preferably for about 20 minutes, and centrifuged at about 40,000 x g for about 10 minutes.

The supernatant is discarded and the precipitate is suspended with a suitable buffer having a pH in the range of about 7.0 to 9.0. Suitable buffers for use in this purification method will have a pH in the range of about 7.0 to about 9.0 and should be cation buffers such as 2-amino-2-(hydroxymethyl)-1,3-propandiol (tris), bis-tris and bis-tris propane, or anionic buffers such as the phosphates like sodium or potassium phosphate. These buffers are known in the biochemical art and are commercially available. The preferred buffer employed

in the suspension of the precipitate is a solution of 25 mM potassium phosphate, 1 mM of [ethylene-bis(oxyethyl-enenitrile)]tetraacetic acid (EGTA), 5 mM of magnesium chloride and 1 mM of DTE.

The solution is dialyzed for approximately 24 hours against the preferred buffer solution as described above typically without the magnesium chloride. One buffer change may be required although more may be employed as needed.

The enzyme preparation is next applied to an anion-exchange chromatography column. Anion-exchange chromatography has two functions. First, cations such as norepinephrine, epinephrine and histamine will not bind to the column. Thus the column helps remove these interfering substances. Secondly, anion-exchange chromatography separates proteins based on their intrinsic ionic nature thus leading to a purified enzyme preparation.

Anion-exchange chromatography includes the use of a column material comprised of an alkylamine, for example a diethylaminoethyl or triethylaminoethyl moiety, covalently attached to any of a variety of matrices such as cellulose or any number of other polymers. A variety of these chromatographic materials are commercially available and the preferred material is sold by Pharmacia Chemicals as diethylaminoethyl-Sephacel.

After the enzyme preparation is applied to the column, the column is eluted with the same buffer as employed above. The buffer eluate typically contains approximately 20% by volume of an alkyldiol such as

glycerol. Elution is continued until all non-adsorbed protein has been eluted. The enzyme activity is then eluted preferably with the same buffer, and the enzyme containing fractions are collected and concentrated by routine methods, such as by ultrafiltration.

The concentrated enzyme preparation is next further purified with molecular size exclusion chromatography. Molecular size exclusion effectively removes smaller molecules capable of decreasing the purity of the enzyme preparation. The support material used should meet two criteria. First, the support material is selected so that it will not retain molecules having a molecular weight above approximately 200,000. Accordingly, high molecular weight impurities come off the column first, and are discarded. Secondly, in regard to the material retained by the column, the support material should have a molecular weight fractionation range from 10-130,000 Daltons. This enables it to facilitate removal of endogenous methyltransferase enzymes from the MAT, since the methyltransferases typically have molecular weights around 30,000 and MAT has a molecular weight on the order of 80,000.

The chromatography support material employed in molecular size exclusion chromatography is readily available, and a preferred support material is Ultrogel ACA-44 commercially available from LKB Corp., Gaithersburg, Maryland. The column is typically equilibrated with a buffer having a pH in the range of about 7.5 to 8.5 prior to the enzyme purification. The column is eluted with buffer and the fractions containing the

enzyme are combined. The preferred buffer is 50 mM potassium TES, 5 mM magnesium chloride, 1 mM EGTA, 10% glycerol by volume and 1 mM DTE to a pH of about 8. As the fractions elute from the column they are typically assayed by any one of several routine methods such as UV spectrometry, enzyme assays and the like in order to localize enzyme activity. The enzyme thus substantially purified is suitable for use in the present enzymatic synthesis.

The reaction mixture containing ATP, L-methionine and substantially purified methionine adenosyltransferase in a suitable buffer is incubated at a temperature in the range of about 20°C to about 40°C for a period of about 30 to 120 minutes. The reaction is preferably conducted at about 37°C for about 60 minutes.

The S-adenosylmethionine thus prepared may be easily isolated by procedures well known to those of ordinary skill in the art. Due to the increased purity of the S-adenosylmethionine prepared by the present process, the procedure for purifying the compound is greatly simplified as compared to existing purification procedures. Typically the reaction mixture is passed over a cation exchange column which retains the product but no unreacted methionine or the S-adenosylhomocysteine byproduct. Finally, dilute hydrochloric acid is passed over the column and the S-adenosylmethionine product is collected. The product thus isolated is typically stored at cold temperatures to prevent decomposition. Further, it is preferred to add ethanol to the mixture for additional stability.

The procedure used to purify the methionine adenosyltransferase provides superior enzyme for use in the present enzymatic synthesis of S-adenosylmethionine because the purified enzyme does not catalyze the formation of S-adenosylmethionine contaminants. These contaminants are believed to be formed by competing methyltransferases which are present in impure preparations of methionine adenosyltransferase. Further, the contaminants are also believed to be various biogenic amines which accept a methyl group from S-adenosylmethionine thereby exhausting the available supply of this valuable reagent. Thus, the use of substantially purified enzyme will permit the synthesis of higher quality S-adenosylmethionine, thereby abolishing the need for extensive isolation procedures of the product.

The following is a proposed procedure for the isolation of tritiated S-adenosylmethionine prepared by the present process at a scale typical of that used for commercial production. This two-step chromatographic approach involves isolation of tritiated S-adenosylmethionine by SP-Sephadex column chromatography and then rapid desalting by Bio-Gel P-2 size exclusion chromatography. R. Glazer and A. Peale (Anal. Biochem. 91, 516-520) have previously reported that cation-exchange chromatography using SP-Sephadex represents an efficient procedure for separation of S-adenosylmethionine from structurally related compounds such as methionine and S-adenosylhomocysteine. Accordingly, following enzymatic synthesis of tritiated S-adenosylmethionine using substantially purified methionine adenosyltransferase,

the reaction may be terminated by adding hydrochloric acid to a final concentration of 0.2M. The mixture is immediately loaded onto a SP-Sephadex column and washed to baseline ($A_{260}$ and radioactivity) with several column volumes of 0.2M HCl. The tritiated S-adenosylmethionine is then specifically eluted with 0.5M hydrochloric acid. The tritiated S-adenosylmethionine is then desalted by size exclusion chromatography using Bio-Gel P-2. This column is typically pre-equilibrated with a dilute mineral acid such as hydrochloric or sulfuric acid and 1-5% Ethanol (v/v). Thus, the product would elute in a chemical environment in which it is maximally stable and suitable for commercial packing. The tritiated S-adenosylmethionine concentration could be adjusted easily by either dilution or concentration under vacuum.

In summary, tritiated S-adenosylmethionine can be isolated by a simple two-step process due to the use of substantially purified methionine adenosyltransferase in the synthetic reaction. In contrast, current commercial processes require extensive "cleanup" of [$^3$H]S-adenosylmethionine, employing, for example, HPLC methodology, in order to produce radioenzymatic grade product. This is a direct result of the use of impure methionine adenosyltransferase preparations which catalyze formation of radiolabeled contaminants which dramatically increase blank values in radioenzymatic assays. The isolation procedure described here is intended to provide an analytical approach to S-adenosylmethionine isolation and is not intended to describe a specific procedure since further optimization may be achieved by subtle changes in column stationary phases or buffer strengths.

Figure 1 and Figure 2 visually illustrate the advantages obtained by employing substantially purified methionine adenosyltransferase in the present process. Further, the figures depict the development of a useful analytical technique for evaluating the purity of the enzyme.

Figure 1 illustrates the relative stability of tritiated S-adenosylmethionine in a preparation of the substantially purified enzyme methionine adenosyltransferase employed in the present process as compared to a typical enzyme preparation currently employed in the synthesis of commercial tritiated S-adenosylmethionine. The procedure used to obtain this representation was as follows. Tritiated S-adenosylmethionine was combined with water, substantially purified enzyme or the enzyme purified by the process of Cantoni in Biochemical Preparations 5, 58 (1955) in separate tubes. The tubes were run in duplicate. Each tube was incubated at approximately 37°C for about 60 minutes and the reaction was terminated by the addition of potassium borate. The resulting aqueous solution was extracted with toluene: isoamyl alcohol (3:2, v:v) and the organic phase was dried. Two aliquots from both the basic aqueous solutions and organic extracts were spotted on a silica gel plate and developed in a solvent system containing 80% chloroform, 17% methanol and 3% of a solution of 70% ethylamine and 30% ammonium hydroxide by volume. The plate was then exposed photographic film for about 16 hours at -70°C and the film was developed according to standard procedures. This procedure is known as fluorescence enhanced autoradiography.

The procedure followed to obtain the results shown in Figure 2 is similar to that provided above. Tritiated L-methionine was combined with either water, an excess of substantially purified enzyme or the enzyme of Cantoni separately. The samples were incubated at 37°C for 30 minutes and the reaction was terminated by the addition of either acid, base or a neutral pH aqueous buffer. Each solution was extracted with toluene:isoamyl alcohol (3:2, v:v) and the organic phases were dried. Each sample was developed by thin layer chromatography using the same solvent system and technique as described for Figure 1.

The results of the studies used in Figures 1 and 2 illustrate the unsatisfactory nature of the enzyme of Cantoni for use in the synthesis of S-adenosylmethionine, and the relative purity of the enzyme employed in the present process. The enzyme prepared according to Cantoni, supra is believed to contain biogenic amines which are converted to methylated products under the conditions specified to other amines containing the radiolabeled methyl group from tritiated S-adenosylmethionine. Further, as a result of this reaction, S-adenosyltransferase inhibitors such as homocysteine are formed, thereby depleting the amount of available methyl donor.

The following procedure illustrates a method for obtaining methionine adenosyltransferase in sufficient purity for use in the present process for preparing S-adenosylmethionine.

Purification Procedure for Methionine Adenosyltransferase

All steps in the following purification scheme were performed at 0°-4°C.

Five Wistar rats (200-300 g) were killed by decapitation and the livers were removed and immediately chilled in ice-cold saline. The tissue (47.1 g) was minced, homogenized with four volumes of isotonic potassium chloride (1.19%) containing 5 mM magnesium chloride using a Brinkmann Polytron and centrifuged at 40,000 x g for 20 minutes. The supernatant was then recentrifuged at 220,000 x g for one hour.

The supernatant was filtered through gauze and adjusted to 1 mM by addition of DTE. The enzyme was then precipitated with solid ammonium sulfate by making the preparation 56% saturated (2.2 M). The precipitate was collected following centrifugation for 10 minutes at 40,000 x g, resuspended in 30 ml of 25 mM potassium phosphate - 1.0 mM EGTA - 5 mM magnesium chloride - 1.0 mM DTE pH 7.5 (Buffer A) and dialyzed for 16 hours against 5 l. of Buffer A without the magnesium chloride.

The dialyzed protein was loaded on a diethylaminoethyl-Sephacel column (7 x 1.6 cm) which had been previously equilibrated with 400 ml of Buffer A containing 20% glycerol. After the 280 nm absorbance had returned to baseline, the column was eluted at a flow rate of approximately one ml/min with a 150 ml linear gradient from 25 mM to 250 mM potassium phosphate. Fractions containing peak enzyme activity were pooled

and concentrated to approximately 10 ml in an Amicon ultrafiltration cell using a pM-10 Diaflo membrane.

The concentrated protein was loaded on an ACA 44 column (90 x 2 cm) which had been previously equilibrated with 50 mM potassium TES - 5 mM magnesium chloride - 1 mM EGTA - 10% glycerol - 1 mM DTE pH 8.0. The column was eluted at a flow rate of 30 ml/hour and 6 ml fractions were collected. Fractions containing peak activity were pooled and concentrated to 10 ml. The enzyme thus substantially purified was suitable for use in the present enzymatic synthesis of S-adenosylmethionine.

An assay has been developed for quantifying the activity of the methionine adenosyltransferase employed in the present process. The assay provides an analytical basis for measurement of methionine adenosyltransferase activity during purification of the enzyme and simultaneously provides the basis for the preparative synthesis of radiolabeled S-adenosylmethionine employing substantially purified enzyme.

Methionine adenosyltransferase activity may be measured radiometrically by coupling the reaction represented by the present process synthesizing radiolabeled S-adenosylmethionine to catechol-O-methyltransferase (COMT) and measuring the conversion of catechol to radiolabeled O-hydroxyanisole. Accordingly, the synthesis of radiolabeled O-hydroxyanisole is a direct function of the S-adenosylmethionine concentration, which is proportional to the amount of methionine adenosyltransferase activity present in the primary

reaction. This assay is based on the biochemical principle represented by the following reaction scheme:

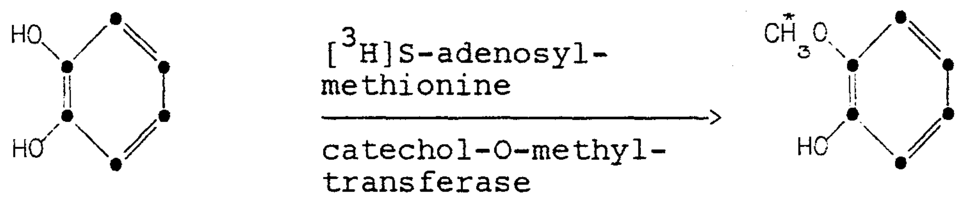


catechol

radiolabeled O-hydroxy-anisole

+ S-adenosyl-homocysteine

The following procedure illustrates the assay described above.

<u>Assay for Methionine Adenosyltransferase</u>

A 75 µl initial reaction mixture contained the following components: 15.0 µmol of potassium TES pH 8.0, 0.5 µmol of EGTA, 0.75 µmol ATP (magnesium salt), 75 nm DTE, and 7.5 nm of L-[methyl-$^3$H]methionine (2.5 µCi; 0.3 Ci/mm). The reaction was initiated by the addition of 25 µl of methionine adenosyltransferase. The second reaction was initiated by the sequential addition of 25 µl of COMT and 10 µl of 11 mM catechol. After incubating for 15 minutes at 37°C the reaction was terminated by the addition of 50 µl of 2.5 M potassium borate pH 11 and 1.25 ml of heptane containing 2.5% (v/v)

isoamyl alcohol. After vortexing and centrifuging at 1500 x g for 5 minutes, a 1 ml aliquot of the organic phase was transferred to a scintillation vial containing 10 ml of Econofluor (an organic counting scintillant from New England Nuclear, Boston, Mass.) and then quantified by liquid scintillation spectrometry.

The starting materials employed in the present process are all known and commercially available. For example, when preparing radiolabeled S-adenosylmethionine, radiolabeled L-methionine must be employed. This material is commercially available from either New England Nuclear or Amerscham. Adenosine 5'-triphosphate is also available commercially from a variety of biochemical supply houses, and published procedures for isolating this compound from muscle are numerous. See, e.g., Berger in Biochim. Biophys. Acta. 20, 23, (1956).

Claims

1. A process for the enzymatic synthesis of S-adenosylmethionine which comprises reacting L-methionine with at least a molar equivalent amount of adenosine 5'-triphosphate in the presence of a catalytic amount of methionine adenosyltransferase in a suitable buffer at a temperature in the range of about 20°C to about 40°C, a substantially purified methionine adenosyltransferase preparation being used as the source of the methionine adenosyltransferase.

2. The process of claim 1 wherein the methionine adenosyltransferase preparation is essentially free of methyl transferases.

3. The process of claim 1 wherein the methionine adenosyltransferase preparation is essentially free of molecules having a molecular weight of less than 30,000.

4. The process of claim 1 wherein the methionine adenosyltransferase preparation is essentially free of molecules having a molecular weight of less than 50,000.

5. A methionine adenosyltransferase preparation that is essentially free of methyl transferases.

6. A methionine adenosyltransferase preparation that is essentially free of molecules having molecular weights of less than 30,000.

7. A methionine adenosyltransferase preparation of claim 9 that is essentially free of molecules having a molecular weights of less than 50,000.

8. A process for preparing a substantially purified methionine adenosyltransferase preparation which comprises subjecting a relatively impure methionine adenosyltransferase preparation to size exclusion chromatography to remove essentially all molecules having molecular weights below 30,000 from the preparation.

## Claims

1. A process for the enzymatic synthesis of S-adenosylmethionine which comprises reacting L-methionine with at least a molar equivalent amount of adenosine 5'-triphosphate in the presence of a catalytic amount of methionine adenosyltransferase in a suitable buffer at a temperature in the range of about 20°C to about 40°C, a substantially purified methionine adenosyltransferase preparation being used as the source of the methionine adenosyltransferase.

2. The process of claim 1 wherein the methionine adenosyltransferase preparation is essentially free of methyl transferases.

3. The process of claim 1 wherein the methionine adenosyltransferase preparation is essentially free of molecules having a molecular weight of less than 30,000.

4. The process of claim 1 wherein the methionine adenosyltransferase preparation is essentially free of molecules having a molecular weight of less than 50,000.

5. A process for preparing a substantially purified methionine adenosyltransferase preparation which comprises subjecting a relatively impure methionine adenosyltransferase preparation to size exclusion chromatography to remove essentially all molecules having molecular weights below 30,000 from the preparation.

FIG. I

**Stability of ³H - S-Adenosylmethionine in Various MAT Enzyme Preparations**

**³H - SAM plus**

| Base | | | Organic Solvent | | |
|---|---|---|---|---|---|
| Water | Substantially Purified Enzyme | Cantoni Enzyme | Water | Substantially Purified Enzyme | Cantoni Enzyme |

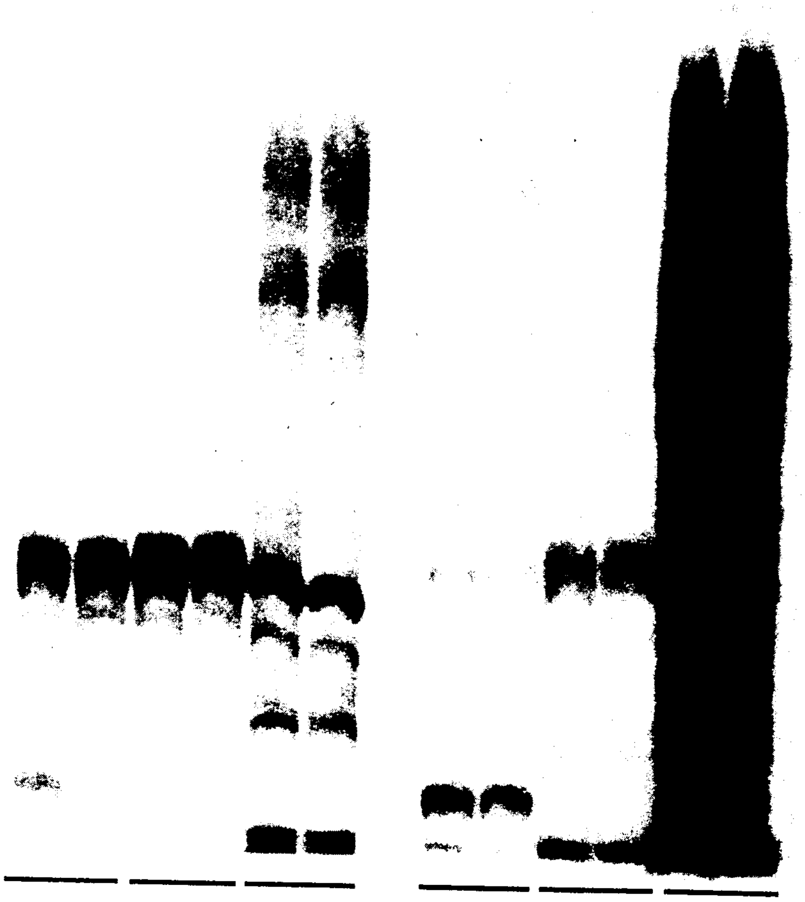

FIG.2

**Stability of $^3H$-Methionine in Various MAT Enzyme Preparations**

**$^3H$ - Methionine plus**